# EUROPÄISCHE PATENTSCHRIFT

(11) **EP 1 490 503 B1**
(45) Veröffentlichungstag und Bekanntmachung des Hinweises auf die Patenterteilung: **20.02.2008**
(21) Anmeldenummer: 03708223.7
(22) Anmeldetag: 12.03.2003
(51) Int. Cl.: C12Q 1/00, C12Q 1/26, C12Q 1/28

(54) **AMPEROMETRISCHER DICKSCHICHT-BIOSENSOR ZUR BESTIMMUNG DER WASSERSTOFFPEROXID-KONZENTRATION IN EINER LÖSUNG UND VERFAHREN ZUR HERSTELLUNG DES SENSORS**
AMPEROMETRIC THICK-FILM BIOSENSOR FOR DETERMINING THE HYDROGEN PEROXIDE CONCENTRATION IN A SOLUTION AND A METHOD FOR PRODUCING SAID SENSOR
BIOCAPTEUR A COUCHE EPAISSE AMPEROMETRIQUE SERVANT A DETERMINER LA CONCENTRATION DE PEROXYDE D'HYDROGENE DANS UNE SOLUTION ET PROCEDE DE PRODUCTION DE CE CAPTEUR

(30) Priorität: 12.03.2002 DE 10212570; 24.05.2002 DE 10224140; 12.08.2002 DE 10237405
(43) Veröffentlichungstag der Anmeldung: 29.12.2004
(73) Patentinhaber: BST BIO SENSOR TECHNOLOGIE GmbH, D-13156 Berlin (DE)
(72) Erfinder: PFEIFFER, Dorothea, 13187 Berlin (DE); SCHÖNFUSS, Dirk, 12524 Berlin (DE); SZEPONIK, Jan, 13187 Berlin (DE); GALL, Ingrid, 16341 Zepernick (DE)
(74) Vertreter: Ziebig, Marlene
(86) Internationale Anmeldenummer: PCT/EP2003/002560
(87) Internationale Veröffentlichungsnummer: WO 2003/076648

(56) Entgegenhaltungen:
- EP-A- 0 230 786
- EP-A- 0 304 933
- EP-A- 0 826 962
- DE-A- 4 241 206
- US-A- 5 695 947
- KIM EUN JU ET AL: "Disposable creatinine sensor based on thick-film hydrogen peroxide electrode system." ANALYTICA CHIMICA ACTA, Bd. 394, Nr. 2-3, 9. August 1999 (1999-08-09), Seiten 225-231, XP001153798 ISSN: 0003-2670
- DATABASE EMBASE [Online] ELSEVIER SCIENCE PUBLISHERS, AMSTERDAM, NL; 1996 WANG J ET AL: "Electrochemical activation of screen-printed carbon strips" Database accession no. EMB-1996099864 XP002249741 & ANALYST 1996 UNITED KINGDOM, Bd. 121, Nr. 3, 1996, Seiten 345-350, ISSN: 0003-2654
- PFEIFFER DOROTHEA ET AL: "Amperometric lactate oxidase catheter for real-time lactate monitoring based on thin film technology." BIOSENSORS & BIOELECTRONICS, Bd. 12, Nr. 6, 1997, Seiten 539-550, XP002249053 ISSN: 0956-5663
- BUTLER K L: "Developing a microsensor array for the monitoring of glucose and ketone levels in diabetics." AAAS ANNUAL MEETING AND SCIENCE INNOVATION EXPOSITION, Bd. 168, 2002, Seite A72 XP001153465 Annual Meeting of the American Association for the Advancement of Science;Boston, MA, USA; February 14-19, 2002
- ROSE ANDREAS ET AL: "Quinoprotein glucose dehydrogenase modified thick-film electrodes for the amperometric detection of phenolic compounds in flow injection analysis." FRESENIUS' JOURNAL OF ANALYTICAL CHEMISTRY, Bd. 369, Nr. 2, 11. Januar 2001 (2001-01-11), Seiten 145-152, XP001153800 ISSN: 0937-0633

## Beschreibung

Die Erfindung betrifft einen preiswerten, einfach und materialsparend zu fertigenden und leicht zu betreibenden amperometrischen Dickschicht-Biosensor zur quantitativen H₂0₂-Bestimmung von niedrigen Konzentrationen und ein Verfahren zur Herstellung dieses Sensors. Der erfindungsgemäße Biosensor erlaubt die H₂0₂-Bestimmung im Konzentrationsbereich von 10 bis 10.000 nM, insbesondere im submikromolaren Bereich.

Vor allem im medizinischen und umweltanalytischen Bereich ist es wünschenswert, über preiswerte und einer Massenfertigung leicht zugängliche Biosensoren zur H₂O₂-Bestimmung im submikromolaren Bereich zu verfügen.

Amperometrische Biosensoren, mit deren Hilfe die Konzentration von bestimmten Stoffen in Lösungen bestimmt werden kann, funktionieren nach dem Grundprinzip, dass die Reaktion des zu bestimmenden Stoffes mit einer biologischen Komponente, oft einem Enzym, mit dem elektrochemischen Detektionsmechanismus kombiniert wird. Dieser enzymatischelektrochemische Detektionsmechanismus ist die Reaktion einer Mediatorsubstanz mit dem Enzym im Endzustand nach seiner Reaktion mit dem zu bestimmenden Stoff in der Weise, dass nach der Reaktion des Enzyms und der Mediatorsubstanz das Enzym wieder in seinem ursprünglichen Zustand vorliegt, während die Mediatorsubstanz in einem oxidierten oder reduzierten Zustand vorliegt und in einer elektrochemischen Reaktion an einer Elektrode wieder in seinen Ausgangszustand überführt werden kann. Der bei dieser elektrochemischen Reaktion durch die Elektrode fließende Strom ist der Konzentration des zu bestimmenden Stoffes proportional und kann gemessen werden. Mit Hilfe einer Kalibration ist somit die quantitative Bestimmung von unbekannten Konzentrationen der zu bestimmenden Stoffe in Lösungen möglich.

Ein derartiges Sensor-Prinzip wird im Patent US 4,882,013 mit Tetrathiafulvalen (TTF) als Mediatorsubstanz in verschiedenen Ausführungsvarianten beschrieben.

Die in US 4,882,013 beschriebene erste Ausführungsvariante des Sensor-Prinzips besteht aus einer Graphitfolien-Scheibe auf einem Substrat, die als Elektrode dient und in ein Glasrohr eingeklebt ist. Ein Draht, welcher mit silberhaltigem Klebstoff an der Rückseite der Graphitfolie befestigt ist, dient der Kontaktierung. Diese Elektrode wird für 2 Stunden in eine TTF-haltige Lösung getaucht und danach 60 Minuten an der Luft getrocknet. Danach wird die Elektrode für 90 Minuten in eine Lösung von 1-Cyclohexyl-3(2-morpholinoethyl) carbodiimidmetho-p-toluensulfonat getaucht, welches als bifunktioneller Ligand für die kovalente Anbindung des Enzyms Glucoseoxidase (GOD) dient. Diese so vorbehandelte Elektrode wird nach Abspülen mit destilliertem Wasser 60 Minuten in eine GOD-Lösung getaucht und ist nach abermaligem Spülen mit einem Phosphat-Puffer einsatzbereit. Der so angefertigte Glucose-Biosensor liefert im Konzentrationsbereich bis 25 mM Glucose ein lineares Stromsignal. Die kleinste mit diesem Sensor gemessene Konzentration beträgt 5 mM Glucose. Die Aufbewahrung erfolgt in einer Phosphat-Puffer-Lösung.

Die zweite Ausführungsvariante besteht aus der oben beschriebenen TTF-modifizierten Elektrode, auf die eine konzentrierte Glucosedehydrogenase-Lösung appliziert wird, welche durch eine mit einem Gummiring befestigte modifizierte Dialyse - Membran auf der Oberfläche gehalten wird. Der so angefertigte Glucose-Biosensor liefert im Konzentrations-bereich bis 10 mM Glucose ein lineares Stromsignal. Die kleinste mit diesem Sensor gemessene Konzentration beträgt 1 mM Glucose.

Eine weitere Ausführungsvariante mit einer verbesserten Enzymimmobilisierung nutzt die Möglichkeit, GOD über seine Kohlenhydratketten aneinander und an die Elektrodenoberfläche zu binden. Dazu wird die oben beschriebene Elektrode 15 Minuten in eine ethanolische Lösung von Hexadecylamin getaucht und danach getrocknet. Die trockene Elektrode wird dann 1 Stunde in eine TTF-Lösung getaucht und wiederum getrocknet. Nach dieser Prozedur wird die Elektrode für 90 Minuten in eine periodatoxidierte GOD Lösung getaucht und danach umgehend in eine Adipin Dihydrazin-haltige Pufferlösung überführt. Nach 30 weiteren Minuten kann die Elektrode mit Wasser gespült und eingesetzt werden oder man bewahrt sie in einer Phosphat-Puffer-Lösung auf. Der so angefertigte Glucose-Biosensor liefert im Konzentrations-bereich bis 15 mM Glucose ein lineares Stromsignal. Die kleinste mit diesem Sensor gemessene Konzentration beträgt 5 mM Glucose.

Es wird weiterhin erwähnt, dass TTF ein geeigneter Mediator für das Enzym L-Aminosäureoxidase ist. Außerdem wird gezeigt, dass ein signifikanter Elektronentransfer über TTF zu einer unbehandelten Glas-Kohle-Elektrode erfolgt, wenn sowohl TTF, GOD sowie Glucose in Lösung vorliegen. Das Cyclovoltammogramm zeigt in einer ca. 20 mM Glucoselösung einen deutlichen Peak.

Der in US 4,882,013 vorgeschlagene Aufbau von Sensoren, d.h. von auf Kohlenstoff basierenden Elektroden, auf denen Enzyme und Mediatoren immobilisiert sind, so dass mit ihnen ohne weitere Zusätze Stoffkonzentrationen in Lösungen bestimmt werden können, bringt wesentliche Nachteile mit sich. Der Aufbau ist kompliziert, sehr zeitaufwendig und damit teuer und für eine Massenproduktion ungeeignet. Mit den resultierenden Sensoren können nur Konzentrationen im Bereich von 1-25 mM bestimmt werden. Die fertigen Sensoren müssen in einer Pufferlösung aufbewahrt werden.

Das Patent WO 98/35053 beschreibt einen Sensor für den Nachweis und die Messung eines Analyten in einer biologischen Flüssigkeit. Der Sensor umfasst zwei Enzyme. Eine Ausführungsform des Sensors misst die Wasserstoffperoxid-Konzentration, wobei hierfür eine thermostabile Peroxidase benutzt wird, welche in einem Redox-Hydrogel immobilisiert ist und auf diese Weise eine sensitive Schicht auf einer Arbeitselektrode bildet. Dieser Sensor beinhaltet weiterhin ein zweites, Wasserstoffperoxid generierendes Enzym, das von der Redox-Hydrogelschicht und der Elektrode isoliert ist. Dieses zweite Enzym (z.B. Glucoseoxidase oder Lactatoxidase) generiert Wasserstoffperoxid in Abhängigkeit von der Anwesenheit eines Analyten (z.B. Glucose oder Lactat) oder einer aus dem Analyten gebildeten Verbindung. Das zweite Enzym kann von der Elektrode isoliert werden, indem eine elektrisch isolierende Schicht zwischen sensitive Schicht und zweite Enzymschicht gebracht wird. Alternativ kann das zweite Enzym in einer anorganischen polymeren Matrix immobilisert werden, was vorzugsweise in einem Sol-Gel-Polymerisationsprozess realisiert wird. Eine weitere Stabilisierung des Enzyms kann durch die gemeinsame Immobilisierung mit einem Polyelektrolyt-Polymer in einer Sol-Gel-Matrix erreicht werden.

WO 98/35053 betrifft das Gebiet thermostabiler EnzymSensoren für unterschiedliche Analyte. Als Beispiele sind Blut-Glucose und Blut-Lactat angeführt. Bei den beschriebenen Sensoren wurde bei einer Temperatur von 37°C eine Abnahme der Empfindlichkeit von 10% festgestellt. Es wird insbesondere auf das Einsatzfeld der in vivo kontinuierlichen Messungen von Blut-Glucose und Blut-Lactat abgezielt.

Der beschriebene Sensor ist eindeutig nicht für die Detektion von Konzentrationen im unteren nanomolaren Bereich geeignet. Die beschriebenen Anwendungen für den Sensor zielen auf eine Konzentration des Analyten im millimolaren Bereich ab. Im Ausführungsbeispiel 15 wird die Empfindlichkeit des Sensors mit 0,8 nA/mM Glucose angegeben und ist damit um etwa 5 Größenordnungen schlechter als die geforderte Empfindlichkeit. Aufgrund der Anordnung des Mehrschichtsystems und der daraus resultierenden großen Schichtdicke der sensitiven Schicht des Sensors können lediglich Ansprechzeiten im Minuten-Bereich erzielt werden. Als besonders nachteilig ist auch das langwierige Herstellungsverfahren des Sensors einzuschätzen (vgl. Ausführungsbeispiel 1).

Aufgabe der Erfindung war es, einen preiswerten, einfach zu fertigenden und somit massenproduktionstauglichen Sensor für die quantitative H₂0₂-Konzentrationsbestimmung im submikromolaren Bereich bereitzustellen, der sich durch eine kurze run in-Phase auszeichnet. Der Sensor soll einfach handhabbar, leicht zu betreiben, robust gegenüber mechanischen Einflüssen, haltbar und ohne großen Aufwand lagerfähig sein. Aufgabe der Erfindung war es auch, ein Verfahren zur Herstellung eines solchen Sensors anzugeben.

Überraschenderweise hat sich gezeigt, dass sich ein solcher H₂0₂-Sensor gemäß den kennzeichnenden Merkmalen des Anspruchs 1 herstellen lässt. Vorteilhafte Ausgestaltungen des Herstellungsverfahrens sind Gegenstand der Unteransprüche.

Zur Herstellung des 2-Elektrodensystems wird im Siebdruckverfahren auf ein Substrat, das vorzugsweise ein übliches Keramiksubstrat, z.B. der Firmen Ceramtec, Kyocera oder Coors ist, eine Platin- oder Goldelektrode als Arbeitselektrode aufgebracht. Als Gegenelektrode wird eine Silberelektrode aufgedruckt. Dazu stehen entsprechende kommerziell erhältliche Platin-Pasten, Gold-Pasten und Silber-Pasten z.B. der Firmen Du Pont, Heraeus, ESL oder Acheson zur Verfügung. Die zugehörigen Kontaktbahnen und Kontaktierungspunkte können z.B. mit einer Silber/Palladium-Paste oder Goldpaste aufgedruckt werden. Auch solche Pasten sind kommerziell erhältlich.

Wird als Substrat ein polymerer Träger, z.B. auf Polyimidbasis oder auf Basis von epoxidharzgetränkten Glasfasergeweben, z.B. FR-4 verwendet, so werden polymere Druckpasten z.B. von den Firmen Acheson oder DuPont zum Aufbringen der Elektroden eingesetzt.

Zum Erreichen der angestrebten Sensitivität ist es erfindungswesentlich, dass die Arbeitselektrode mittels einer Pt- oder Au-Paste hergestellt wird. Im Gegensatz dazu wird üblicherweise aufgrund der großen Oberfläche Kohlepaste als Elektrodenmaterial oder aufgrund seiner elektrochemischen (große elektrochemische Überspannung für die elektrochemische Sauerstoff- und Wasserstoffentwicklung) und chemischen Eigenschaften (Möglichkeit der chemischen Immobilisierung von Enzymen auf der Elektrodenoberfläche) Kohlenstoff als Elektrodenmaterial verwendet.

Auf die nicht mit den Elektroden bedruckten Flächen des Substrates und auf die aufgebrachten Kontaktbahnen (aber nicht auf die Kontaktierungspunkte) wird als elektrisch isolierendes Material eine Lackschicht aufgedruckt. Sie dient der Isolation der Leiterbahnen.

Das so hergestellte substratbasierte 2-Elektrodensystem aus Arbeits- und Gegenelektrode, den Kontaktbahnen und Kontaktierungspunkten und der Lackschicht ist auch kommerziell erhältlich und wird gemäß der Erfindung zum hochsensitiven Biosensor zur H₂O₂-Bestimmung modifiziert.

Erfindungsgemäß wird nun die aufgedruckte Silberelektrode vor Aufbringen einer Polymerschicht, die Mediator und Enzym immobilisiert, elektrochemisch modifiziert. Dazu wird in einer chloridhaltigen Lösung ein anodischer Strom durch die Silberelektrode geleitet, wobei sich diese mit einer dünnen Silberchloridschicht überzieht. Dadurch wird erreicht, dass die Referenzelektrode beim Messprozess ein nahezu konstantes Potential behält. Druckt man dagegen, wie es nahe liegt, eine silberchloridhaltige Paste auf, um eine potentialstabile Elektrode zu erzeugen, so wird die gewünschte Sensitivität des Biosensors nicht erreicht.

Die Immobilisierung von Mediator und Enzym unter Einbeziehung eines Polymers kann wie folgt durchgeführt werden. Bevorzugt wird die einen Mediator und ein Enzym enthaltende Polymerschicht auf die Elektroden aufgebracht. Die Polymerschicht wird in der Weise hergestellt, dass eine Lösung oder Suspension, die Polymer, Mediator und Enzym enthält, gleichmäßig sowohl auf die Arbeitselektrode als auch auf die Gegenelektrode und den Zwischenraum zwischen den Elektroden oder nur auf die Arbeitselektrode aufgebracht und eintrocknen gelassen wird. Vorzugsweise sollte das Aufbringen und Trocknen bei einer Temperatur von 18-35°C erfolgen, bevorzugt bei 20 bis 25°C. Alternativ können Mediator und Enzym in getrennten Schichten unter Verwendung eines Polymers in Lösung und/oder als Suspension auf die Elektrode immobilisiert werden. Bevorzugt liegt der Mediator in einer Lösung vor, auf die dann das Enzym in einer Polymerschicht aufgebracht wird.

Als Mediatoren werden Tetrathiafulvalen (TTF) oder seine Derivate, wie z.B. Mono- und Polycarbonsäurederivate oder Mono- und Polyaminoderivate, Ferrocen, Hexacyanoferrat, N-Methylphenazinium (NMP⁺), Methylacridinium (NMA) oder 7,7,8,8-Tetracyanparachinodimethan (TCNQ) eingesetzt.

Als Polymer finden erfindungsgemäß bevorzugt Polyurethane, z.B. alipatische Polycarbonatdiolurethane, alipatische Polyesterurethane, alipatische Polyetherurethane, aromatische Polyesterurethane, Mischungen dieser Urethane oder Mischungen dieser Urethane mit Acrylaten wie z.B. mit anionischen Acrylaten, nichtionischen Acrylaten, anionischen Methacrylaten und modifizierten anionoschen Acrylaten Verwendung. Auch Polyacrylate können eingesetzt werden.

Als Enzym finden Peroxidasen Verwendung. In einer erfindungsgemäß bevorzugten Ausführungsform ist das Enzym Meerrettich-Peroxidase oder Sojabohnen-Peroxidase. Es ist nicht erforderlich, dass das Enzym thermostabil ist.

Je nach eingesetztem Polymer, Mediator und Enzym wird eine Lösung oder eine Suspension hergestellt, welche alle drei Komponenten enthält. Dazu werden zunächst Lösungen des Enzyms und des Mediators hergestellt. Für die Lösung des Enzyms und des Mediators eignen sich, je nach dem, welches Enzym und welcher Mediator verwendet werden, Wasser, Ethanol, Essigsäureethylester, Dimethylsulfoxid (DMSO), Dimethyl-formamid (DMF) und Mischungen aus diesen Lösungsmitteln. Diese Lösungen werden mit einer wässrigen Suspension des Polymers vermischt. Als vorteilhaft hat es sich erwiesen, zunächst z.B. eine wässrige Peroxidaselösung mit einer wässrigen aliphatischen Polyurethan-Suspension zu mischen und dieses Gemisch wiederum mit einer essigsäureethyl-esterhaltigen alkoholischen TTF-Lösung zu vermischen. Bevorzugt werden Enzym-, Mediator- oder Polymerkon-zentrationen von 0,5 - 2% eingesetzt. Für Enzym und Mediator sind Konzentrationen von 1 - 1,5% besonders bevorzugt. Die Konzentration des Polymers sollte in einer besonders bevorzugten Ausführungsform 1,5 - 2% betragen.

Es hat sich gezeigt, dass der gemäß dem erfindungsgemäßen Verfahren hergestellte H₂O₂-Biosensor eine run-in Zeit von weniger als 30 Minuten hat und sich H₂O₂-Konzentrationen im nanomolaren Bereich zuverlässig messen lassen. Die Herstellung des Grundsensors auf der Basis des Siebdruckes in Dickschichttechnologie ist ein für eine Massenproduktion geeignetes und preiswertes Verfahren. Die Immobilisierung des Enzyms und des Mediators mit Hilfe eines Polymers erfolgt zeitsparend in einem Schritt und lässt sich ebenfalls automatisieren. Der Sensor ist ohne besondere Hilfsmittel lange haltbar und bei geringer Größe gut lagerbar.

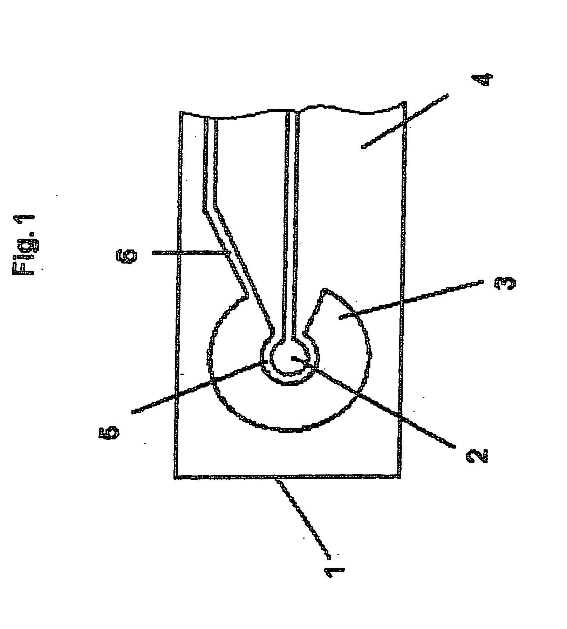

Gegenstand der vorliegenden Erfindung ist daher auch ein Biosensor mit den Merkmalen des Anspruchs 8. Vorteilhafte Ausgestaltungen des Biosensors sind Gegenstand der Unteransprüche. In einer bevorzugten geometrischen Ausgestaltung ist das Substrat (Sensorkörper) 1 planar und der Sensor weist die in den Figuren 1, 3 und 4 dargestellte geometrische Ausgestaltung auf, wobei die Elektroden 2,3 eine runde, eckige oder ovale Form besitzen, vorzugsweise eine runde oder ovale. Die Größe des Substrats 1 beträgt erfindungsgemäß von 2 bis 400 mm², bevorzugt von 4 bis 40 mm².

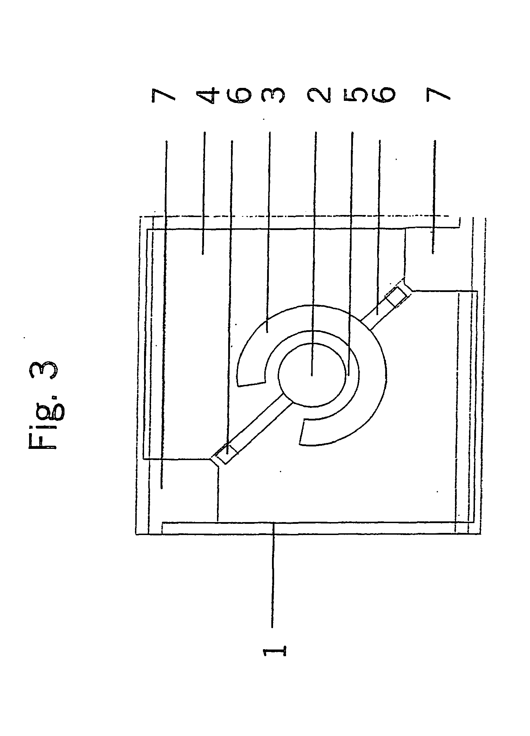

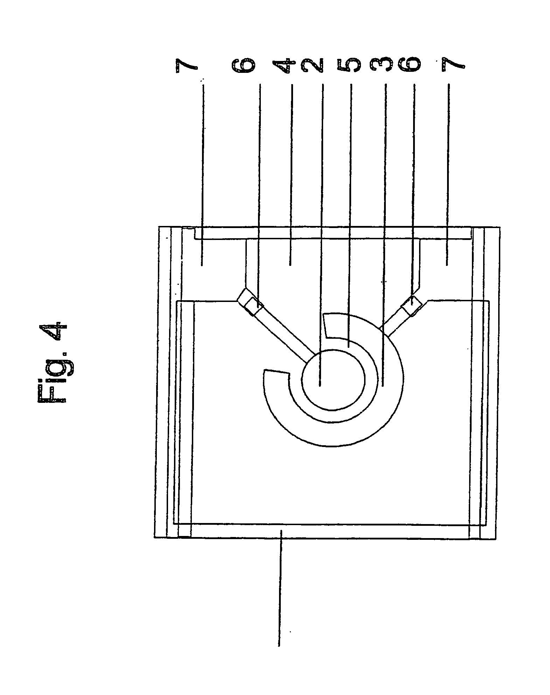

Die in Fig. 3 und 4 dargestellten geometrischen Ausgestaltungen sind insbesondere unter dem Aspekt der Materialeinsparung und der kostensparenden Herstellung vorteilhaft. Während in den Sensorvarianten der Figuren 1, 3 und 4 Arbeits- und Gegenelektrode 2,3 nahezu gleich angeordnet sind, führen die Kontaktbahnen 6 in der Ausgestaltung gemäß Fig. 3 von der Arbeits- und der Referenzelektrode 2,3 zu diagonal angeordneten Kontaktpunkten 7, die sich in gegenüberliegenden Ecken des rechteckigen Sensorkörpers 1 befinden. Alternativ, wie in Fig. 4 dargestellt, können die Kontaktpunkte 7 auch auf einer Seite des Sensorkörpers 1 (quasi parallel zueinander) angeordnet sein, zu denen dann die jeweiligen Kontaktbahnen 6 führen. Der erfindungsgemäße Sensor kann zum besseren Handling wahlweise in eine Halterung eingebracht werden.

In einer weiteren vorteilhaften Ausgestaltung enthält die Polymerschicht des erfindungsgemäßen Biosensors das Enzym, den Mediator und das Polymer im Verhältnis (w/v) von 0,25 - 4 : 0,25 - 4 : 1, vorzugsweise von 0,5 - 1,3 : 0,5 - 1,3 : 1.

Zum Betrieb des Sensors wird dieser bevorzugt in einer Durchflusszelle mit einem Strömungskanal so fixiert, dass der Biosensor eine Wand des Strömungskanals bildet und die mit der sensitiven Polymerschicht bedeckte Arbeitselektrode und ein Teil der Gegenelektrode von der Probe überspült werden. Der Fluss durch die Messzelle wird -durch eine möglichst pulsationsarme Pumpe gewährleistet. Mit Hilfe des Potentiostaten wird zwischen Arbeitselektrode und Gegenelektrode eine Spannung angelegt und der resultierende Strom gemessen. Die Messsignale des Potentiostaten können mit analogen oder digitalen Aufzeichnungsgeräten, z.B. einem y-t-Schreiber, aufgezeichnet werden. Das Grundsignal des Sensors erhält man, wenn man eine H₂O₂-freie Lösung durch die Messzelle pumpt. Wird eine H₂O₂-haltige Lösung durch die Messzelle gepumpt, erhält man ein der Analytkonzentration entsprechendes Sensorsignal.

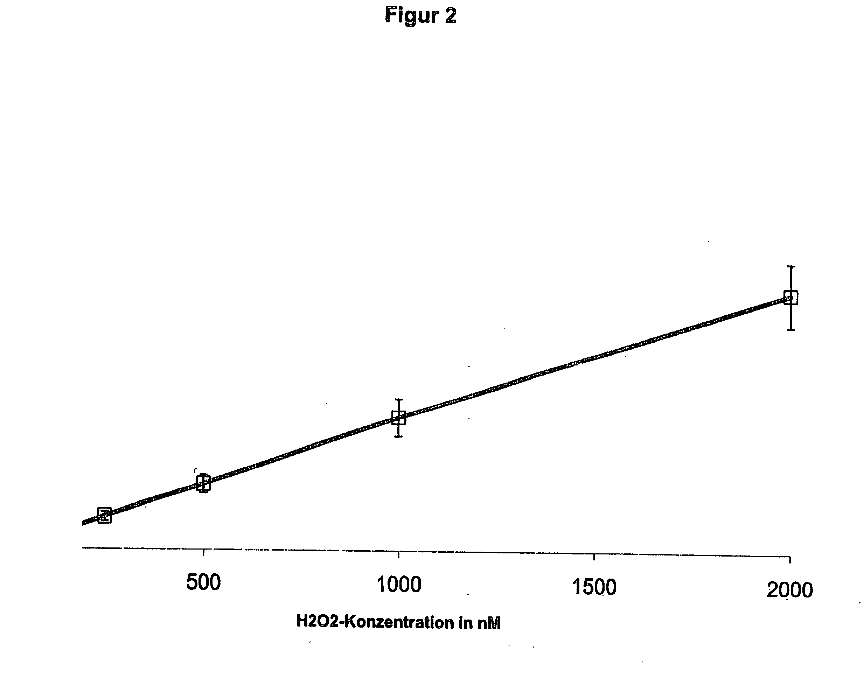

Die mit zehn erfindungsgemäßen Biosensoren ermittelten Messwerte bei H₂O₂-Konzentrationen von 15 nM bis 2.000 nM gemäß Fig. 2 zeigen, dass die Abhängigkeit des Biosensorsignals in diesem Bereich der H₂O₂-Konzentration linear ist und damit der Biosensor leicht kalibrierbar ist.

Untersuchungen zur Haltbarkeit des Sensors haben gezeigt, dass bei einer trockenen Lagerung im Kühlschrank bei 4°C nach 3 Monaten kein signifikanter Empfindlichkeitsverlust auftritt.

Figuren 1, 3 und 4 zeigen den erfindungsgemäßen Biosensor in bevorzugten Ausführungsformen.

Es bedeuten:
1 - Substrat (Sensorkörper)
2 - Platin- oder Goldarbeitselektrode
3 - Ag/AgCl-Referenzelektrode
4 - Lackschicht
5 - Zwischenraum zwischen den Elektroden
6 - Kontaktbahnen der Elektroden
7 - Kontaktpunkte

Fig. 2 zeigt die durchschnittlichen Messwerte, die mit zehn erfindungsgemäßen Biosensoren (Arbeitselektrode Platin, Gegenelektrode Ag/AgCl, Keramiksubstrat, Enzym Peroxidase, Mediator TTF, Polymer Polyurethan) erhalten wurden. Es wurden Probelösungen unterschiedlicher H₂O₂-Konzentrationen von 15 nM bis 2.000 nM in 0,033 M chloridhaltigem Phospatpuffer (pH=7,1) vermessen. Das an die Arbeitselektrode angelegte Potential betrug - 100mV vs. Ag/AgCl.

## Patentansprüche

1. Verfahren zur Herstellung eines amperometrischen Biosensors zur Bestimmung der Wasserstoffperoxid-Konzentration unter Verwendung eines dickschichttechnologisch hergestellten 2-Elektrodensystems auf einem Substrat und Aufbringen eines Enzyms und eines Mediators auf die Elektroden.
wobei ein im Siebdruckverfahren hergestelltes ; 2-Elektrodensystem verwendet wird; das auf' einem Substrat eine Platin- oder Goldelektrode als Arbeitselektrode, eine Silberelektrode als Gegenelektrode, deren Kontaktbahnen und Kontaktierungspunkte, und eine elektrisch isolierende Lackschicht aufweist, die die nicht mit den Elektroden bedruckten Flächen des Substrats und die aufgebrachten Kontaktbahnen bedeckt, die Silberelektrode des 2-Elektrodensystems elektrochemisch mit einer Silberchloridschicht überzogen wird, indem in einer chloridhaltigen Lösung ein anodischer Strom durch die Silberelektrode geleitet wird, danach ein Polymer, der Mediator und das Enzym in einer Lösung oder einer Suspension oder in getrennten Lösungen und/oder getrennten Suspensionen homogen auf die Elektroden und: deren Zwischenraum oder nur auf die Arbeitselektrode aufgebracht und getrocknet wird.

2. Verfahren nach Anspruch 1,
**dadurch gekennzeichnet, dass**
als Mediator Tetrathiafulvalen (TTF) oder dessen Derivate verwendet werden.

3. Verfahren nach Anspruch 1,
**dadurch gekennzeichnet, dass**
als Polymer ein Polyurethan oder ein Polyacrylat oder eine Mischung aus diesen Polymeren verwendet wird.

4. Verfahren nach Anspruch 1,
**dadurch gekennzeichnet, dass**
als Enzym eine Peroxidase verwendet wird.

5. Verfahren nach Anspruch 1,
**dadurch gekennzeichnet, dass**
die Lösung oder Suspension das. Enzym, den Mediator und das Polymer in einer Konzentration von je 0,5 - 2% enthält.

6. Verfahren nach Anspruch 1,
**dadurch gekennzeichnet, dass**
das Aufbringen und Trocknen der den Mediator, das Enzym und das Polymer enthaltenden Lösung oder Suspension bei einer Temperatur von 18-35°C vorgenommen wird.

7. Amperometrischer Dickschicht-Biosensor zur Bestimmung der Wasserstoffperoxid-Konzentration in einer Lösung, umfassend ein Substrat (1), eine auf das Substrat aufgedruckte Platin- oder Gold-Arbeitselektrode (2)', eine aufgedruckte Silber-Gegenelektrode (3), die mit einer Silberchloridschicht bedeckt ist, eine isolierende Lackschicht (4), die die nicht mit den Elektroden (2,3) bedruckten Flächen des Substrats (1) und die Kontaktbahnen (6) der Elektroden bedeckt, eine homogene Polymerschicht, die ein Enzym und einen Mediator enthält und die Elektroden (2,3) und deren Zwischenraum (5) bedeckt.

8. Biosensor nach Anspruch 7,
**dadurch gekennzeichnet, dass**
die Polymerschicht aus einem Polyurethan oder einem Polyacrylat oder einer Mischung aus diesem Polymeren besteht.

9. Biosensor nach Anspruch 7,
**dadurch gekennzeichnet, dass**
das in der Polymerschicht enthaltene Enzym eine Peroxidase ist.

10. Biosensor nach Anspruch 7,
**dadurch gekennzeichnet, dass**
der in der Polymerschicht enthaltene Mediator Tetrathiafulvalen (TFF) oder dessen Derivate sind.

11. Biosensor nach Anspruch 7,
**dadurch gekennzeichnet, dass**
die Polymerschicht das Enzym, den Mediator und das Polymer im Verhältnis (w/v) von 0, 25 - 4 : 0,25 - 4 : 1, vorzugsweise von 0,5 - 1,3 : 0,5 - 1,3 : 1, enthält.

12. Biosensor nach Anspruch 7,
**dadurch gekennzeichnet, dass**
das Substrat (1) eine Größe von 2 bis 400 mm² aufweist, vorzugsweise von 4 bis 40 mm².

13. Biosensor nach Anspruch 7,
**dadurch gekennzeichnet, dass**
die Elektroden (2,3) eine runde, ovale oder eckige Form besitzen, vorzugsweise eine runde oder ovale.

14. Biosensor nach Anspruch 7,
**dadurch gekennzeichnet, dass**
die Kontaktbahnen 6 und die Kontaktpunkte 7 so angeordnet sind, dass sich die beiden Kontaktpunkte 7 auf einer Diagonalen jeweils in der entgegengesetzten Ecke des Sensorkörpers 1 befinden.

15. Biosensor nach Anspruch 7,
**dadurch gekennzeichnet, dass**
die Kontaktbahnen 6 und die Kontaktpunkte 7 so angeordnet sind, dass die beiden Kontaktpunkte 7 sich an einer Seite des Sensorkörpers 1 (parallel zueinander) befinden.

## Claims

1. A method for the production of an amperometric biosensor for the determination of the concentration of hydrogen peroxide using a 2-electrode system, which is produced using thick-film technology, on a substrate and placing an enzyme and a mediator on the electrodes, said method using a 2-electrode system produced in a screen printing process, which system has, on a substrate, a platinum or gold electrode as working electrode, a silver electrode as counterelectrode, the contact paths and contact points thereof, and an electrically insulating lacquer layer covering the areas of substrate not printed with electrodes and the contact paths applied thereto, the silver electrode of the 2-electrode system being coated electrochemically with a silver chloride layer by passing an anodic current through the silver electrode in a solution containing chloride, followed by homogeneously applying a polymer, said mediator and said enzyme in solution or suspension or in separate solutions and/or separate suspensions to the electrodes and the space therebetween or solely to the working electrode and drying.

2. The method according to claim 1,
**characterized in that**
tetrathiafulvalene (TTF) or derivatives thereof are used as mediator.

3. The method according to claim 1,
**characterized in that**
a polyurethane or a polyacrylate or a mixture of these polymers is used as polymer.

4. The method according to claim 1,
**characterized in that**
a peroxidase is used as enzyme.

5. The method according to claim 1,
**characterized in that**
the solution or suspension includes the enzyme, the mediator and the polymer at a respective concentration of 0.5 to 2%.

6. The method according to claim 1,
**characterized in that**
applying and drying the solution or suspension containing the mediator, the enzyme and the polymer is effected at a temperature of 18-35°C.

7. An amperometric thick-film biosensor for the determination of the hydrogen peroxide concentration in a solution, comprising a substrate (1), a platinum or gold working electrode (2) printed on the substrate, a printed silver counterelectrode (3) covered with a silver chloride layer, an insulating lacquer layer (4) covering the areas of substrate (1) not printed with electrodes (2,3) and the contact paths (6) of the electrodes, a homogeneous polymer layer which contains an enzyme and a mediator and covers the electrodes (2,3) and the space (5) therebetween.

8. The biosensor according to claim 7,
**characterized in that**
the polymer layer consists of a polyurethane or a polyacrylate or a mixture of these polymers.

9. The biosensor according to claim 7,
**characterized in that**
the enzyme included in the polymer layer is a peroxidase.

10. The biosensor according to claim 7,
**characterized in that**
the mediator included in the polymer layer is tetrathiafulvalene (TFF) or a derivative thereof.

11. The biosensor according to claim 7,
**characterized in that**
the polymer layer includes the enzyme, the mediator and the polymer at a ratio (w/v) of 0.25 - 4 : 0.25 - 4 : 1, preferably 0.5 - 1.3 : 0.5 - 1.3 : 1.

12. The biosensor according to claim 7,
**characterized in that**
the substrate (1) has a size of from 2 to 400 mm², preferably from 4 to 40 mm².

13. The biosensor according to claim 7,
**characterized in that**
the electrodes (2,3) are round, oval or angular in shape, preferably round or oval.

14. The biosensor according to claim 7,
**characterized in that**
the contact paths 6 and contact points 7 are arranged in such a way that each of the two contact points 7 is situated on a diagonal in opposite corners of the sensor body 1.

15. The biosensor according to claim 7,
**characterized in that**
the contact paths 6 and contact points 7 are arranged in such a way that the two contact points 7 are situated on one side of the sensor body 1 (in parallel).

## Revendications

1. Procédé de fabrication d'un biocapteur ampérométrique destiné à déterminer la concentration en peroxyde d'hydrogène en utilisant un système à 2 électrodes fabriqué par une technologie de film épais sur un substrat et en appliquant une enzyme et un médiateur sur les électrodes,
dans lequel on emploie un système à 2 électrodes fabriqué par un procédé de sérigraphie, qui comprend sur un substrat une électrode de platine ou d'or à titre d'électrode de travail, une électrode d'argent en tant que contre-électrode, leurs pistes de contact et leurs points de mise en contact, et une couche de vernis électriquement isolante qui recouvre les surfaces du substrat non empreintes avec les électrodes et les pistes de contact appliquées, on revêt l'électrode d'argent du système à 2 électrodes par électrochimie d'une couche de chlorure d'argent, en faisant passer dans une solution chlorurée un courant anodique au travers de l'électrode d'argent, on applique ensuite un polymère, le médiateur et l'enzyme dans une solution ou une suspension ou des solutions séparées et/ou des suspensions séparées de manière homogène sur les électrodes et leur espace intermédiaire ou uniquement sur l'électrode de travail et on sèche.

2. Procédé selon la revendication 1,
**caractérisé en ce**
**qu'**on utilise, en tant que médiateur, du tétrathiafulvalène (TTF) ou ses dérivés.

3. Procédé selon la revendication 1,
**caractérisé en ce**
**qu'**on utilise, en tant que polymère, un polyuréthane ou un polyacrylate ou un mélange de ces polymères.

4. Procédé selon la revendication 1,
**caractérisé en ce**
**qu'**on utilise, en tant qu'enzyme, une peroxydase.

5. Procédé selon la revendication 1,
**caractérisé en ce que**
la solution ou la suspension contient l'enzyme, le médiateur et le polymère à une concentration pour chaque de 0,5 à 2 %.

6. Procédé selon la revendication 1,
**caractérisé en ce**
**qu'**on procède à l'application et au séchage de la solution ou la suspension contenant le médiateur, l'enzyme et le polymère à une température de 18 à 35°C.

7. Biocapteur à film épais ampérométrique destiné à la détermination de la concentration de peroxyde d'hydrogène dans une solution, comprenant un substrat (1), une électrode de travail de platine ou d'or (2) empreinte sur le substrat, une contre-électrode d'argent empreinte (3), qui est recouverte avec une couche de chlorure d'argent, une couche de vernis isolante (4) qui recouvre les surfaces du substrat (1) non empreintes avec les électrodes (2,3) et les pistes de contact (6) des électrodes, une couche de polymère homogène, qui contient une enzyme et un médiateur et recouvre les électrodes (2,3) et leur espace intermédiaire (5).

8. Biocapteur selon la revendication 7,
**caractérisé en ce que**
la couche de polymère est constituée d'un polyuréthane ou d'un polyacrylate ou d'un mélange de ces polymères.

9. Biocapteur selon la revendication 7,
**caractérisé en ce que**
l'enzyme contenue dans la couche de polymère est une peroxydase.

10. Biocapteur selon la revendication 7,
**caractérisé en ce que**
le médiateur contenu dans la couche de polymère est du tétrathiafulvalène (TTF) ou ses dérivés.

11. Biocapteur selon la revendication 7,
**caractérisé en ce que**
la couche de polymère contient l'enzyme, le médiateur et le polymère dans un rapport (poids/volume) de 0,25 - 4 : 0,25 - 4 : 1, de préférence de 0,5 - 1,3 : 0,5-1,3 : 1.

12. Biocapteur selon la revendication 7,
**caractérisé en ce que**
le substrat (1) a une grandeur de 2 à 400 mm², de préférence de 4 à 40 mm².

13. Biocapteur selon la revendication 7,
**caractérisé en ce que**
les électrodes (2,3) ont une forme ronde, ovale ou polygonale, de préférence ronde ou ovale.

14. Biocapteur selon la revendication 7,
**caractérisé en ce que**
les pistes de contact 6 et les points de contact 7 sont disposés de telle manière que les deux points de contact 7 se trouvent sur une diagonale respectivement, aux deux coins opposés du corps du capteur 1.

15. Biocapteur selon la revendication 7,
**caractérisé en ce que**
les pistes de contact 6 et les points de contact 7 sont disposés de telle manière que les deux points de contact 7 se trouvent apposés à un côté du corps du capteur 1 (en parallèle).
